(19) Europäisches Patentamt
European Patent Office
Office européen des brevets

(11) Veröffentlichungsnummer: **0 332 073**
**A1**

(12) **EUROPÄISCHE PATENTANMELDUNG**

(21) Anmeldenummer: **89103745.9**

(22) Anmeldetag: **03.03.89**

(51) Int. Cl.⁴: **C07D 405/06 , C07D 405/14 , A01N 43/50**

(30) Priorität: **10.03.88 DE 3807951**

(43) Veröffentlichungstag der Anmeldung:
**13.09.89 Patentblatt 89/37**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Karbach, Stefan, Dr.**
**Grundwiesenweg 44**
**D-6730 Neustadt(DE)**
Erfinder: **Janssen, Bernd, Dr.**
**Leuschnerstrasse 18 a**
**D-6700 Ludwigshafen(DE)**
Erfinder: **Kober, Reiner, Dr.**
**Im Schlittweg 20**
**D-6701 Fussgoenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **Fungizide Imidazolylmethyloxirane.**

(57) Imidazolylmethyloxiran der allgemeinen Formel I

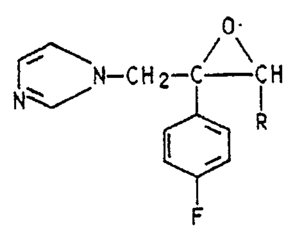

I,

in welcher R Phenyl, Pyridyl, Tetrahydropyranyl, Norbornyl, Cycloalkyl oder Cycloalkenyl bedeutet, wobei diese Reste gegebenenfalls substituiert sind oder dessen für Pflanzen verträgliches Säureadditionssalz oder Metallkomplex und diese Verbindungen enthaltende Fungizide.

EP 0 332 073 A1

## Fungizide Imidazolylmethyloxirane

Die vorliegende Erfindung betrifft neue Imidazolverbindungen, Verfahren zu ihrer Herstellung und diese enthaltende Fungizide.

Es ist bekannt, Azolylmethyloxirane, die durch Halogenphenyl substituiert sind, als Fungizide zu verwenden (DE-3 218 130.2, DE-3 536 529.3). Ihre fungizide Wirkung ist jedoch unbefriedigend.

Es wurde nun gefunden, daß Verbindungen der allgemeinen Formel I

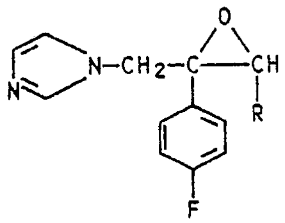

I,

in welcher R Phenyl, Pyridyl, Tetrahydropyranyl, Norbornyl, $C_3$-$C_{12}$-Cycloalkyl oder $C_5$-$C_8$-Cycloalkenyl bedeutet, wobei diese Reste gegebenenfalls einfach bis dreifach durch Halogen, Nitro, Phenoxy, Alkyl, Amino, Alkoxy, Haloalkoxy oder Halogenalkyl mit jeweils 1 bis 4 Kohlenstoffatomen substituiert sind oder deren für Pflanzen verträgliche Säureadditionssalze oder Metallkomplexe eine bessere fungizide Wirkung, insbesondere gegen Botrytiskrankheiten, besitzen als die bekannten Triazolverbindungen.

Die Verbindungen der Formel I enthalten chirale Zentren und werden im allgemeinen Form von Racematen bzw. als Diastereomerengemische von erythro- bzw. threo-Formen erhalten. Die erythro- bzw. threo-Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel kann man sowohl die einheitlichen Diastereomere bzw. Enantiomere wie auch deren bei der Synthese anfallende Gemische verwenden. Die vorliegende Erfindung umfaßt sowohl die Einzelverbindungen als auch deren Gemische.

Bevorzugt werden die Imidazolylmethyloxirane der Formel I, in der R Phenyl bedeutet, welches unsubstituiert ist oder durch einen oder zwei Substituenten aus der Gruppe Fluor, Chlor, Brom oder Trifluormethyl substituiert ist. Besonders bevorzugt werden Verbindungen, in denen R Fluorphenyl oder Chlorphenyl bedeutet.

R bedeutet beispielsweise: Phenyl, Halogenphenyl, 2-Chlorphenyl, 2-Fluorphenyl, 2-Bromphenyl, 3-Chlorphenyl, 3-Bromphenyl, 3-Fluorphenyl, 2,6-Difluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxyphenyl, 4-Trifluormethoxyphenyl, 2,4-Dimethoxyphenyl, Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, 3-Trifluoromethylphenyl, 4-Trifluoromethylphenyl, Cyclopropyl, Cyclobutyl, Cyclopentyl, Cyclohexyl, Cyclooctyl, Cyclododecyl, 4-Tetrahydropyranyl, 2-Cyclohexenyl, 3-Cyclohexenyl, Norbornyl, 3-Pyridyl. Säureadditionssalze sind die Salze mit anorganischen oder organischen Säuren beispielsweise die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die erfindungsgemäßen Wirkstoffsalze werden hergestellt durch Umsetzung der Imidazolylmethyloxirane (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die Imidazolylmethyloxirane mit entsprechenden Metallsalzen umsetzt, z.B. mit Kupfersulfat, Zinkchlorid, Zinnchlorid.

Die Verbindungen der Formel I können z.B. hergestellt werden, indem man

a) eine Verbindung der Formel II

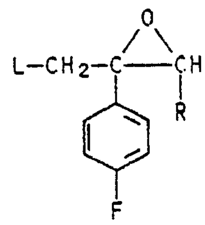

II,

in welcher R die oben angegebene Bedeutung hat und L eine nucleophil substituierbare Abgangsgruppe (Halogen, OH) darstellt, mit einer Verbindung der Formel III

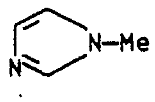

III,

in der Me ein Wasserstoffatom, ein Metallatom (Na, K) oder eine Trimethylsilylgruppe bedeutet, zur Umsetzung bringt, oder

b) eine Verbindung der Formel IV

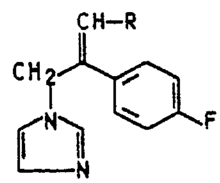

IV,

in welcher R die angegebene Bedeutung hat, in die Epoxide überführt. Die Reaktion a) erfolgt - falls Me ein Wasserstoffatom bedeutet - gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quarternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150 °C, drucklos oder unter Druck, kontinuierlich oder diskontinuierlich durchgeführt.

Ist Me ein Metallatom, dann wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120 °C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und Sulfolan.

Geeignete Basen die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- und Kalium-tert.-butoxid, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B.

Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100° C, vorzugsweise bei 20 bis 80° C. Bei Anwesenheit eines Lösungsmittels wird zweckmäßigerweise beim Siedepunkt des jeweiligen Lösungsmittels gearbeitet.

Die neuen Ausgangsverbindungen II erhält man durch Epoxidierung der entsprechenden Olefine V:

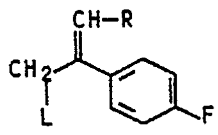

V,

(vgl. G. Dittus in Houben-Weyl-Müller, Methoden der Organischen Chemie, Georg Thieme Verlag, Stuttgart, 1965, Bd. VI, 3, Seite 385 ff).

Die Verbindung V stellt man her, indem man Olefine der Formel VI

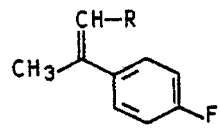

VI

nach bekannten Methoden in Allylposition halogeniert oder oxidiert.

Geeignete Halogenierungsreagenzien sind z.B. N-Chlor- und N-Bromsuccinimid in halogenierten Kohlenwasserstoffen wie Tetrachlorkohlenstoff, Trichlorethan oder Methylenchlorid bei Temperaturen zwischen 20 und 100° C. Zur Allyloxidation verwendet man z.B. Perester wie Perbenzoesäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100° C.

Die so erhaltenen Allylhalogenide bzw. -alkohole V werden anschließend in die entsprechenden Epoxide II (L = Halogen, 0H) übergeführt. Dazu oxidiert man die Olefine V mit Peroxycarbonsäuren, wie Perbenzoesäure, 3-Chlorbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat. Man arbeitet zwischen 10 und 100° C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30° C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide II (L = Halogen) gemäß Verfahren a) weiter umgesetzt werden können, überführt man die entsprechenden Epoxialkohole II (L = 0H) in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663,671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die folgenden Beispiele erläutern die Herstellung der Wirkstoffe.

I. Herstellung der Ausgangsstoffe

Vorschrift A

Zu einer Lösung von 85,5 g 2-Trifluormethylbenzaldehyd in 300 ml Methanol werden 8,4 g Natriumhydroxid in 40 ml Wasser gegeben. Das Reaktionsgemisch wird auf 10 °C gekühlt und schnell 69 g 4-Fluorphenylacetaldehyd zugetropft, wobei die Temperatur in der Lösung 30 °C nicht übersteigt. Nach zweistündigem Rühren bei Raumtemperatur (20 °C) wird der farblosen Reaktionslösung 300 ml Wasser zugesetzt und die entstandene Emulsion mit Methyl-tert.-butylether ausgeschüttelt. Die organische Phase wird abgetrennt, über Natriumsulfat getrocknet und im Vakuum eingedampft. Bei der anschließenden Destillation des verbleibenden Rückstandes werden bei 0,3 mbar und 116 °C Übergangstemperatur 96 g (65 %) E-2-(4-Fluorphenyl)-3-(2-trifluormethylphenyl)-propenal erhalten.

Vorschrift B

96 g E-2-(4-Fluorphenyl)-3-(2-trifluormethylphenyl)-propenal werden in 300 ml Methanol gelöst und 2,3 ml Natronlauge (konz.) zugesetzt. Die Reaktionslösung wird bei 0 °C gerührt, während 27,7 g Wasserstoffperoxid (ca. 50 %ig) langsam zugetropft werden, wobei die Innentemperatur von 30 °C nicht überschritten wird. Nach beendeter Zugabe wird sechs Stunden bei Raumtemperatur gerührt und anschließend 5 g Natriumborhydrid zugegeben, das in wenig 10 %iger Natronlauge gelöst war. Nachdem das Reaktionsgemisch 18 Stunden bei Raumtemperatur gerührt worden war, wird der Lösung 100 ml Wasser zugesetzt und die entstandene Emulsion mit Methylenchlorid ausgeschüttelt. Die isolierte organische Phase wird daraufhin über Natriumsulfat getrocknet und eingeengt. Man erhält 90 g (89 %) cis-2-Hydroxymethyl-2-(4-fluorphenyl)-3-(2-trifluormethylphenyl)-oxiran.

Vorschrift C

Zu einer Lösung von 90 g cis-2-Hydroxymethyl-2-(4-fluorphenyl)-3-(2-trifluormethylphenyl)-oxiran in 300 ml Methylenchlorid und 58 g Triethylamin werden bei Raumtemperatur 61 g 4-Methylbenzolsulfonsäurechlorid zugesetzt. Nach 24 Stunden wird das Reaktionsgemisch mit wässriger Natriumhydrogencarbonat-Lösung und Wasser gewaschen, über Natriumsulfat getrocknet und im Vakuum eingedampft. Aus dem Rückstand erhielt man 128,4 g (95 %) cis-2-(4-Methylphenylsulfonyloxymethyl)-2-(4-fluorphenyl)-3-(2-trifluormethylphenyl)-oxiran.

II. Herstellung der Endprodukte

Beispiel 1

27,3 g Imidazol und 9,5 g Natriumhydrid (80 %ige Dispersion in Mineralöl) wurden in 300 ml N,N-Dimethylformamid suspendiert und bei Raumtemperatur mit einer Lösung aus 128,4 g cis-2-(4-Methylphenylsulfonyloxymethyl)-2-(4-fluorphenyl)-3-(2-trifluormethylphenyl)-oxiran in 200 ml N,N-Dimethylformamid versetzt. Nach acht Stunden wurde die Reaktionslösung auf Wasser gegeben und mit Methyl-tert.-butylether extrahiert. Die organische Phase wurde mit Wasser gewaschen, über Natriumsulfat getrocknet und das Lösungsmittel im Vakuum eingedampft. Man erhielt aus Methyl-tert.-butylether/n-Hexan 94 g cis-2-Imidazolylmethyl-2-(4-fluorphenyl)-3-(2-trifluormethyl-phenyl)-oxiran mit dem Schmelzpunkt 127 - 135 °C (Verbindung Nr. 1)

Entsprechend Beispiel 1 können die in der Tabelle aufgeführten Verbindungen hergestellt werden.

Tabelle

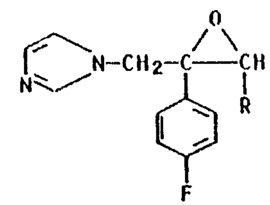

| Nr. | R | Isomer | Schmp. |
|---|---|---|---|
| 1 | $2-CF_3-C_6H_4$ | cis | 127-135°C |
| 2 | $3-CF_3-C_6H_4$ | cis | |
| 3 | $4-CF_3-C_6H_4$ | cis | |
| 4 | $2-OCH_3-C_6H_4$ | cis | Harz |
| 5 | $3-OCH_3-C_6H_4$ | cis | |
| 6 | $4-OCH_3-C_6H_4$ | cis | |
| 7 | $2-Cl-C_6H_4$ | cis | 119-122°C |
| 8 | $3-Cl-C_6H_4$ | cis | Harz |
| 9 | $4-Cl-C_6H_4$ | cis | |
| 10 | $2,4-Cl_2-C_6H_3$ | cis | |
| 11 | $2-Cl-4-F-C_6H_3$ | cis | 135°C |
| 12 | $2,3-Cl_2-C_6H_3$ | cis | |
| 13 | $2,5-Cl_2-C_6H_3$ | cis | |
| 14 | $2,6-Cl_2-C_6H_3$ | cis | |
| 15 | $2-Cl-6-F-C_6H_3$ | cis | |
| 16 | $2-F-C_6H_4$ | cis | |
| 17 | $3-F-C_6H_4$ | cis | |
| 18 | $4-F-C_6H_4$ | cis | |
| 19 | $2-Br-C_6H_4$ | cis | 122-124°C |
| 20 | $3-Br-C_6H_4$ | cis | |

| Nr. | R | Isomer | Schmp. |
|---|---|---|---|
| 21 | 4-Br-$C_6H_4$ | cis | |
| 22 | 3-$NO_2$-$C_6H_4$ | cis | |
| 23 | 4-$NO_2$-$C_6H_4$ | cis | |
| 24 | 3-$NH_2$-$C_6H_4$ | cis | |
| 25 | 4-$NH_2$-$C_6H_4$ | cis | |
| 26 | 2-Cl-5-$NO_2$-$C_6H_3$ | cis | |
| 27 | 2,4-$OCH_3$-$C_6H_3$ | cis | |
| 28 | 3,4-$OCH_3$-$C_6H_3$ | cis | |
| 29 | 4-$C_2H_5$-$C_6H_4$ | cis | |
| 30 | 4-tert.butyl-$C_6H_4$ | cis | |
| 31 | 4-O-tert.butyl-$C_6H_4$ | cis | |
| 32 | 4-O-Phenyl-$C_6H_4$ | cis | |
| 33 | 3-O-Phenyl-$C_6H_4$ | cis | |
| 34 | 3-Pyridyl | cis | |
| 35 | Cyclopropyl | cis | |
| 36 | Cyclobutyl | cis | |
| 37 | Cyclopentyl | cis | |
| 38 | Cyclohexyl | cis | IR: 2928,2853,1510,1450,1227,838 $cm^{-1}$ |
| 39 | 3-Cyclohexenyl | cis | |
| 40 | 3-Cyclopentenyl | cis | |
| 41 | Norbornyl | cis | |
| 42 | 4-tetrahydropyranyl | cis | |
| 43 | 3-tetrahydropyranyl | cis | |
| 44 | $C_6H_5$ | cis | |
| 45 | 4-$OCF_3$-$C_6H_4$ | cis | Harz |
| 46 | 2,6-$F_2$-$C_6H_3$ | cis | 182-188°C |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,

Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen
, Plasmopara Viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine oder Amide (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 7 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 11 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 11 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 7 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 11 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 7 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 11 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 7 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,

5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoffe werden cis-2-(1,2,4-Triazol-1-yl-methyl)-2-(4-chlorphenyl)-3-phenyl-oxiran (A) - bekannt aus DE-3 218 130.2 - und cis-2-(1,2,4-Triazol-1-ylmethyl)-2-(4-fluorphenyl)-3-(2-chlorphenyl)-oxiran (B) - bekannt aus DE-3 536 529.3 - verwendet.

Anwendungsbeispiel 1

Wirksamkeit gegen Plasmopara viticola

Blätter von Topfreben der Sorte "Müller Thurgau" wurden mit wäßriger Spritzbrühe, die 80 % (Gew.%) Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Danach wurden die Blätter mit einer Zoosporenaufschwemmung von Plasmopara viticola (Rebenperonospora) infiziert. Dann wurden die Reben zunächst für 48 Stunden in einer wasserdampfgesättigten Kammer bei 24° C und anschließend für 5 Tage in einem Gewächshaus mit Temperaturen zwischen 20 und 30° C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 7 und 11 bei der Anwendung als 0,05 %ige wäßrige Spritzbrühen eine bessere fungizide Wirkung (90 %) zeigen als der bekannte Vergleichswirkstoff A (50 %).

Anwendungsbeispiel 2

Wirksamkeit gegen Botrytis cinerea

Kleinparzellen von Freiland-Erdbeerpflanzen der Sorte "Senga-Sengana" wurden zum Zeitpunkt der Hauptblüte behandelt. 2 Tage später wurde die gesamte Anlage mit einer Sporensuspension von Botrytis cinerea inoculiert und nach weiteren 5 Tagen wurden die entsprechenden Parzellen nochmals behandelt. 38 Tage danach wurden die reifen Früchte auf den Botrytis-Befall durchgesehen.

Das Ergebnis zeigt, daß der Wirkstoff 7 bei einer Aufwandmenge von 2 kg Wirkstoff/ha eine bessere fungizide Wirkung zeigt (73 %) als der bekannte Vergleichswirkstoff B (0 %).

## Ansprüche

1. Imidazolylmethyloxirane der allgemeinen Formel I

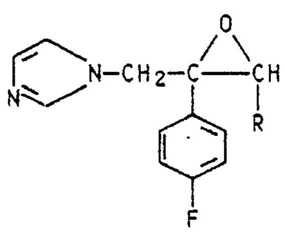

I,

in welcher R Phenyl, Pyridyl, Tetrahydropyranyl, Norbornyl, $C_3$-$C_{12}$-Cycloalkyl oder $C_5$-$C_8$-Cycloalkenyl bedeutet, wobei diese Reste gegebenenfalls durch Halogen, Nitro, Phenoxy, Alkyl, Amino, Alkoxy, Haloalkoxy oder Halogenalkyl mit jeweils 1 bis 4 Kohlenstoffatomen substituiert sind sowie deren für Pflanzen verträgliche Säureadditionssalze oder Metallkomplexe.

2. Verbindung der Formel I gemäß Anspruch 1, worin R die Phenylgruppe bedeutet, welche unsubstituiert ist oder durch einen oder zwei Substituenten aus der Gruppe Fluor, Chlor, Brom oder Trifluormethyl substituiert ist.

3. Verbindung der Formel I gemäß Anspruch 1, worin R Fluorphenyl oder Chlorphenyl ist.

4. Fungizides Mittel, enthaltend ein Imidazolylmethyloxiran der allgemeinen Formel I

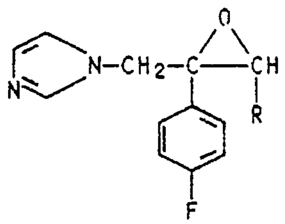

I,

in welcher R Phenyl, Pyridyl, Tetrahydropyranyl, Norbornyl, $C_3$-$C_{12}$-Cycloalkyl oder $C_5$-$C_8$-Cycloalkenyl bedeutet, wobei diese Reste gegebenenfalls durch Halogen, Nitro, Phenoxy, Alkyl, Amino, Alkoxy, Haloalkoxy oder Halogenalkyl mit jeweils 1 bis 4 Kohlenstoffatomen substituiert sind oder dessen für Pflanzen verträgliches Säureadditionssalz oder Metallkomplex und einen inerten Zusatzstoff.

5. Verfahren zur Bekämpfung von Pilzen, dadurch gekennzeichnet, daß man eine fungizid wirksame Menge eines Imidazolmethyloxirans der allgemeinen Formel I

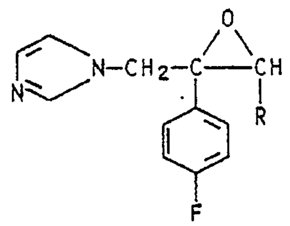

I,

in welcher R Phenyl, Pyridyl, Tetrahydropyranyl, Norbornyl, $C_3$-$C_{12}$-Cycloalkyl oder $C_5$-$C_8$-Cycloalkenyl bedeutet, wobei diese Reste gegebenenfalls durch Halogen, Nitro, Phenoxy, Alkyl, Amino, Alkoxy, Haloalkoxy oder Halogenalkyl mit jeweils 1 bis 4 Kohlenstoffatomen substituiert sind oder dessen für Pflanzen verträgliches Säureadditionssalz oder Metallkomplex auf die Pilze oder auf durch Pilzbefall bedrohte Materialien, Flächen, Pflanzen oder Saatgüter einwirken läßt.

6. Verbindung gemäß Anspruch 1, dadurch gekennzeichnet, daß R 2-Chlorphenyl bedeutet und die Verbindung in der cis-Form vorliegt.

7. Verbindung gemäß Anspruch 1, dadurch gekennzeichnet, daß R 2-Chlor-4-fluorphenyl bedeutet und die Verbindung in der cis-Form vorliegt.

Europäisches Patentamt

# EUROPÄISCHER RECHERCHENBERICHT

Nummer der Anmeldung

EP 89 10 3745

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 196 038 (BASF AG) * Ansprüche 1,3-5; Seite 6, Verbindungen 8,23; Seite 11, Verbindung 38 *; & DE - A - 35 36529 (Kat. D) | 1-7 | C 07 D 405/06<br>C 07 D 405/14<br>A 01 N 43/50 |
| A | EP-A-0 094 564 (BASF AG) * Ansprüche 1,5-7 *; & DE - A - 32 18130 (Kat. D) | 1,4,5 | |
| A | EP-A-0 089 100 (IMPERIAL CHEMICAL INDUSTRIES PLC) * Ansprüche 1-6,41-43 * | 1,4,5 | |
| | | | **RECHERCHIERTE SACHGEBIETE (Int. Cl.4)** |
| | | | C 07 D 405/00<br>C 07 D 521/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 23-05-1989 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)